# EUROPEAN PATENT SPECIFICATION

(11) **EP 1 059 356 B1**
(45) Date of publication and mention of the grant of the patent: **02.11.2005**
(21) Application number: 00401568.1
(22) Date of filing: 05.06.2000
(51) Int. Cl.: C12N 15/86, C12N 5/10, A61K 48/00

(54) **Replicating retroviral constructs, preparation and uses for gene delivery**
Replizierende retrovirale Konstrukte, ihre Herstellung und Verwendungen für den Gentransfer
Constructions rétrovirales capables de la réplication autonome, leur préparation et leurs utilisations pour le transfert de gènes

(30) Priority: 09.06.1999 EP 99401391
(43) Date of publication of application: 13.12.2000
(73) Proprietor: UNIVERSITE PIERRE ET MARIE CURIE PARIS VI, 75252 Paris Cédex 05 (FR)
(72) Inventor: Klatzmann, David, 75013 Paris (FR); Morel, Arnaud, 92310 Sevres (FR); Holzer, Georg, 75005 Paris (FR); Salzmann, Jean-Loup, 75005 Paris (FR)
(74) Representative: Becker, Philippe

(56) References cited:
- WO-A-98/02529
- US-A- 5 858 740
- US-A- 5 869 035
- PETROPOULOS C. J. ET AL.: "REPLICATION-COMPETENT RETROVIRUS VECTORS FOR THE TRANSFER AND EXPRESSION OF GENE CASSETTES IN AVIAN CELLS" JOURNAL OF VIROLOGY, vol. 65, no. 7, 1991, pages 3728-3737, XP000579739 ISSN: 0022-538X
- GREENHOUSE J. J. ET AL.: "HELPER-INDEPENDENT RETROVIRUS VECTORS WITH ROUS-ASSOCIATED VIRUS TYPE O LONG TERMINAL REPEATS" JOURNAL OF VIROLOGY, vol. 62, no. 12, page 4809-4812 XP000579738 ISSN: 0022-538X
- GUNDLACH B. R. ET AL.: "Construction, replication and immunogenic properties of a simian immunideficiency virus expresing IL-2" JOURNAL OF VIROLOGY, vol. 3, no. 71, March 1997 (1997-03), pages 2225-2232, XP002078381 ISSN: 0022-538X
- HOATLIN M. E. ET AL.: "AMPLIFIED AND TISSUE-DIRECTED EXPRESSION OF RETROVIRAL VECTORS USING PING-PONG TECHNIQUES" JOURNAL OF MOLECULAR MEDICINE, vol. 73, 1995, pages 113-120, XP000770505 ISSN: 0946-2716

## Description

This invention relates to compositions and methods for the delivery of nucleic acids into cells, in vitro, ex vivo or in vivo. More preferably, the invention relates to compositions and methods for the delivery of nucleic acids into cells using replicating viral constructs, in particular replicating retroviral constructs. The invention also relates to the preparation of said constructs, genetically modified cells which contain said constructs, such as packaging cells, as well as the use of these compositions and methods for the delivery of any selected polynucleotide to a cell, for experimental, prophylactic, therapeutic or diagnostic applications.

The delivery of nucleic acids to cells finds applications in various biotechnology and pharmacology areas, such as experimental research, clinical research, therapeutic or prophylactic treatment as well as diagnostic. Gene delivery in vitro can be used to produce recombinant proteins or viruses, for instance, as well as to produce stable recombinant cells for screening purposes. In vivo or ex vivo, gene delivery enables the construction of transgenic animals, the study of gene regulation, as well as therapeutic and/or prophylactic treatments of mammals, including human beings. In this regard, increasing amounts of animal and clinical trials have been reported in the literature, designed at delivering to subjects nucleic acids encoding therapeutic or antigenic polypeptides. These approaches use different gene transfer strategies, which comprise for instance the use of (i) viral vectors (or cells producing viral vectors), (ii) non-viral vectors (such as plasmids in combination with chemical agents or physical treatments), (iii) naked DNA or (iv) genetically modified cells which are grafted into the patient, optionally upon encapsulation to limit immune reaction.

The use of recombinant retroviruses is one of the most successful methods to introduce a nucleic acid into dividing cells, in vitro, in vivo or ex vivo. This gene transfer strategy has proven to be useful for both basic research and clinical applications. In this regard, in humans, much progress has been made for the transduction of hematopoietic progenitors (Nolta et al., Exp. Hematol. 20 (1992) 1065-1071), mature lymphocytes (Bunnell et al., Proc. Natl. Sci. USA **92** (1995) 7739-7743), tumor cells (Klatzmann et al. Hum. Gen. Ther. 9 (1998) 2595-2604; Caruso et al.; PNAS 90 (1993) 7024-7028), etc. Retroviruses have thus been validated as a gene delivery vehicle in vivo, in human subjects, for clinical applications (treatment of cancers, genetic diseases (e.g., ADA-deficiency, SCID), viral infections (e.g., HIV treatment), immune disorders (e.g., graft versus host disease, autoimmune diseases, etc.), etc.

In particular, Hughes et al (Journal of Virology, 61, 3004-3012, 1987) discloses a recombinant replicating retroviral genome comprising a CAT polynucleotide inserted downstream of the *env* gene and in which a splice acceptor site is inserted between the *env* gene and the CAT polynucleotide. It relates to "subgroup D vectors" having a tropism for mammalian cells.

Recombinant retroviruses which have been used in the art for gene delivery applications, are genetically modified to be rendered defective, i.e., to avoid replication of their genome and/or propagation upon infection of competent cells, in the absence of trans-comptementing functions. In this respect, most recombinant retroviruses are created by replacing, in the recombinant genome, the viral genes gag, pol and env with a nucleic acid of interest. The recombinant, defective retroviruses are prepared in a so-called packaging cell, which produces the complementing functions encoded by gag, pol and env. Examples of such packaging cell lines are, for instance PA317 (Miller et Buttimore, Mol. Cell. Biol. 6 (1986) 2895), PsiCRIP (Danos et Mulligan, PNAS 85 (1988) 6460), or GP+EnvAm12 (Markowitz et al. Virology 167 (1988) p. 400). Other examples of retrovirus packaging cells have been described for instance in EP 243 204, WO89/07150, WO90/02806, US5,766,945, EP476,953, WO93/04167 and WO93/10218. The recombinant retroviruses produced in said cells are infectious, but cannot replicate themselves upon infection. In this regard, they are considered replication-defective. Such replication-defective recombinant retroviruses have been constructed using different types of retroviruses, including MoMLV (Moloney Murine Leukemia Virus) ALV, BLV, MMTV or RSV for instance, or using lentiviruses such as HIV, SIV or CAEV, for instance.

It is generally admitted that the gene delivery vectors should be as deficient as possible in order to avoid adverse effects upon administration in vivo. For this reasons, the retroviral vectors which have been used to date essentially lack all of the viral proteins gag, pol and env. The same concem also exists for all other types of viral vectors which are currently being used, such as adenoviral vectors, AAV, herpes virus vectors and the like. For instance, adenoviruses generally comprise a deletion of the E1 region at least, and current efforts are being made to produce "gutless" adenovectors, i.e., adenoviral vectors devoid of all viral coding sequences. Similarly, most recombinant AAV vectors are devoid of the rep and cap coding regions.

However, while much progress has now been made in gene delivery vectors, there is still a need for alternative strategies which may increase gene transfer efficacy, gene expression stability and/or facilitate gene vector production, especially for industrial uses.

In Patent Application n° PCT/FR 95/00208, Applicants have disclosed a novel gene delivery concept. This concept comprises delivering to a cell, in vitro, ex vivo or in vivo, nucleic acid construct(s) comprising all of the genetic elements allowing said cell to produce a recombinant virus. This method therefore uses nucleic acids that create in situ recombinant virus producing cells. This method has been used for instance to deliver therapeutic or toxic genes in vivo, or to prepare vaccine compositions (see PCT/FR 97/00619 Noguiez-Hellin et al., PNAS 93 (1996) 4175-4180). This method offers several advantages over prior gene delivery vectors, and in particular it avoids the need for packaging cells, allows the use of recombinant plasmids to deliver the viral genes in vivo, provides efficient gene transfer in vivo, etc.

The instant invention now provides a novel approach for gene delivery into cells. This approach is based on the use of replicating viral constructs to deliver genes in vitro, ex vivo or in vivo. In contrast with all prior existing methods, which where based on the use of replication defective viral constructs, the instant invention now stems from a new and original concept of using replicating viral constructs, in particular replicating retroviral constructs, for in vivo, ex vivo or in vitro delivery of polynucleotides. The instant invention shows that such constructs can be made in high quantity and quality and efficiently deliver and express any gene or nucleic acid of interest into cells in vitro, ex vivo or in vivo.

The instant invention therefore relates to methods and compositions for polynucleotide delivery into cells, in vitro, ex vivo or in vivo. More particularly, the invention relates to compositions comprising replicating viral constructs and their use in delivering polynucleotides to cells. In other aspects, the invention also resides in uses of packaging cells which produce replicating recombinant viruses.

### The use of replicating viral constructs

As indicated above, in one embodiment, the invention resides in replicating viral constructs and uses thereof, in particular replicating retroviral constructs and uses thereof.

Numerous systems of retroviral vectors have been developed for gene delivery and gene therapy in the past decade. The main advantage of these systems is seen in the ability of these vectors to stably integrate into the hosts genome. In this regard, retroviruses such as MoMLV, selectively infect dividing cells, and are therefore considered as promising vectors for the transduction of proliferating cells (i.e., tumor cells, proliferating lymphocytes, etc.). Alternatively, retroviruses such as lentiviruses can infect also non-dividing cells and thus can be used as vectors to deliver genes to quiescent cells. For instance, MoMLV-derived retroviral vectors can be used to deliver genes to proliferating cells such as tumor cells or hematopoietic cells (in particular activated T lymphocytes), with the objective of destroying or modifying said cells. Lentivirus-derived vectors may be used to deliver polynucleotides to quiescent cells such as fibroblasts or muscle cells (in particular smooth or skeletic muscle), especially with the objective of raising an immune response against specific antigens or groups of antigens. Depending on the target cells or treatment conditions, these vectors, however, do not always provide sufficient transduction efficiency, and several approaches have been developed in order to address this aspect (strong promoters, targeted vectors, ex vivo gene transfer, etc.). The instant invention now provides a novel approach to improve the transduction efficiency of retroviral vectors. This approach is based on the use of replicating-viruses, which retain the ability to spread in the target cell population, tissue or organ.

Within the context of the present invention, the term "replicating" means that the construct(s) which are being used contain the genetic elements necessary for production of replicating recombinant viruses, i.e., recombinant viruses which are capable of replicating in the absence of any trans-complementing function, and therefore to spread in the target cell population, tissue or organ. These recombinant viruses are also termed replication-competent.

It is known that the genomic organization of retroviruses comprises essentially the following elements:
- a LTR ("Long Terminal Repeat") region, located at each end of the genome, and functioning as the origin of replication and transcriptional promoter region. Each LTR region is composed essentially of three functional regions termed U3, R and U5, U5 and U3 being involved in the provirus integration,
- a packaging sequence ("Psi"), involved in the packaging of the proviral genome in the viral particle,
- three coding regions, designated gag, pol and env, coding the core proteins (gag), the enzymes (reverse transcriptase, protease, integrase) and the envelope glycoprotein (env).

In a general way, a replicating retroviral construct according to the present invention is therefore any construct (e.g., a nucleic acid, a plasmid, a virus) comprising at least functional gag, pol and env genes, as well as a polynucleotide to be delivered to cells. More specifically, the replicating retroviral construct according to the present invention comprises (i) functional gag, pol and env genes, (ii) a polynucleotide to be delivered to cells, (iii) a retroviral packaging sequence and (iv) at least one retroviral LTR sequence. These elements are also referred to in this application as the recombinant, replication-competent retroviral genome.

With regard to lentivirus, their genome further comprises additional coding or regulatory sequences, such as vif, vpr, vpu, vpx, rev, tat, and nef. A replicating lentivirus-type retroviral construct of the instant invention would thus preferably comprise elements (i) - (iv) listed above as well as (v) functional vif, vpr, vpu, vpx, rev, tat and nef sequences, or only a part thereof necessary for replication of the viral genome.

A replicating retroviral construct according to the present invention is therefore any construct (e.g., a nucleic acid, a plasmid, a virus) comprising at least a replication-competent retroviral genome, as defined above.

Within the context of the present invention, the expression "polynucleotide" designates any nucleic acid molecule whose delivery to a cell, culture, tissue, organ or organism is desired, as will be discussed below. This term may also encompass retroviral genes, such as retroviral envelope genes with fusogenic activity for instance. In this embodiment, the polynucleotide would represent the env gene.

More preferably, the replication-competent retroviral genome is a DNA or RNA molecule comprising, in the 5' -> 3' order:
(i) a retroviral 5' LTR region
(ii) a retroviral packaging sequence
(iii) functional gag, pol and env genes, and
(iv) a retroviral 3' LTR region,
said genome further comprising a selected polynucleotide inserted in a region of said genome which do not prevent the replication capacity of said genome.

Elements (i) to (iv) may be prepared by known techniques, starting from various materials and different types of retroviruses.

In particular, the functional gag and pol genes, the LTR sequence and the packaging region can derive from (be obtained from the genome of) retroviruses such as MoMLV (Moloney Murine Leukemia Virus) ALV, BLV, MMTV or RSV for instance, or from lentiviruses such as HIV, SIV or CAEV, for instance. These elements can be isolated and manipulated following techniques well known to the skilled artisan, or isolated from plasmids available in the art.

The functional env gene may encode an ecotropic (infectious in cells of the same species) or amphotropic (infectious in various species) envelope. Particular envelopes that can be used in the instant invention are, for instance, the envelope of the following viruses : 4070A (Ott et al., J. Virol. Vol. 64 (1990) p757-766), RD114, 10A1, VSV, VIH, rabies virus or GALV (Delassus S. et al., Virology 173 (1989) 205-213, which is fusogenic, or derivatives or other types disclosed in EP 99400964.5, which are fusogenic or non fusogenic). The envelope may also be of cellular origin, such as a membrane protein allowing targeting of the retrovirus to a selected ligand, such as a CD4 receptor for instance. Preferably, the envelope is a retroviral envelope having tropism for mammalian cells, more preferably human cells, in particular an amphotropic or retargeted envelope. GALV, 4070A or 10A1 represent preferred embodiment for the construction of replicating viruses of the instant invention. In this regard, as will be discussed below, the envelope gene or any variant thereof may also represent the polynucleotide of the vector constructs.

In addition, elements (i) and (iv) may be further modified in order to provide improved transduction efficiency, expression stability, or control over any spread of the retrovirus in vitro, ex vivo or in vivo. In particular, in preferred embodiments, modified envelope protein comprising a targeting moiety (a ligand, receptor, etc.) and/or a selected epitope, for instance, are being used.These modifications or variants will be disclosed in more detail below.

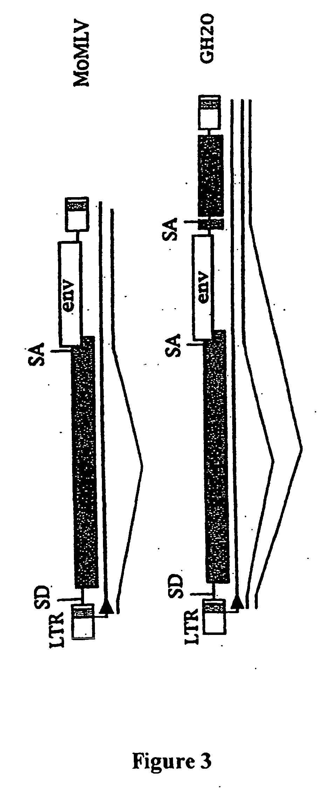

In a first embodiment of the present invention, the polynucleotide is inserted 3' of the envelope gene, preferably in the same transcriptional orientation as the env gene. In this respect, the invention now discloses a preferred and efficient way of producing a functional recombinant replication-competent genome as described above, comprising the creation of a second splice acceptor site within the viral genome, allowing translation of the polynucleotide from the transcriptional promoter contained in the LTR region. An example of such a construct is represented on Figure 3. In a preferred embodiment, the invention therefore resides in a retroviral construct comprising a replication-competent retroviral genome comprising the following elements, in the 5' -> 3' order:
(i) a retroviral 5' LTR region
(ii) a retroviral packaging sequence
(iii) functional gag, pol and env genes,
(iv) a polynucleotide, and
(v) a retroviral 3' LTR region.
the retroviral genome further comprising a splice acceptor site between the env gene and the polynucleotide.

Where lentivirus-type retroviral constructs are used, elements (iii) above further comprises functional coding or regulatory sequences selected from vif, vpr, vpu, vpx, rev and tat, which are necessary for replication of such viruses.

This type of constructs has proven to be advantageous since it allows efficient expression of the polynucleotide, with no need for an additional (internal) promoter region. Furthermore, such an artificial construct, providing expression of the polynucleotide by alternative splicing, mimicks the natural expression mode in complex retroviruses. The inserted second acceptor splice site may duplicate the acceptor splice site already present in the retroviral construct, upstream of the env gene. This particular embodiment is illustrated in the Examples, wherein the created acceptor splice site comprises a fragment of a retroviral DNA of below 1 kb, comprising the MoMLV splice acceptor site. In a particular embodiment, the created splice acceptor site comprises a fragment of less than 1 kb, preferably less than 0,6 kb, of a sequence corresponding to the sequence of a retroviral acceptor splice site or a variant thereof, retaining the activity of a splice acceptor site. Variants may be tested for their activity in a retroviral construct of the instant invention, by verifying expression of the polynucleotide. In this respect, the structure and general properties of splice acceptor sites, in particular retroviral splice acceptor sites, are known to the skilled artisan. Splice acceptor sites are found in the genome of all retroviruses, between the pol and env genes. These splice acceptor sites regulate transcription of the env gene from the 5'-LTR. Any splice acceptor site can therefore be used in the instant invention, such as a DNA fragment of a different size containing the splice acceptor, a synthetic sequence, or a corresponding region from another retrovirus. In a preferred embodiment, the splice acceptor site comprises all or a portion of SEQ ID NO:6, or a variant thereof. More preferably, the splice acceptor site is a nucleic acid fragment comprising the sequence of position 437 to position 802 of SEQ ID NO: 6, or a variant thereof. Even more preferably, the inserted nucleic acid comprises the sequence of position 532 to position 540 of SEQ ID NO: 6, or a variant thereof. In preferred embodiments the inserted nucleic acid comprising the splice acceptor site comprises less than about 1,5 kb, even more preferably, about 1kb or less.

Furthermore, as another aspect of this invention, the duplication of a retroviral (e.g., the MoMLV) splice acceptor region results in a tandem repeat, flanking the env gene (Figure 3). Deletion of genomic sequences due to repeated sequences in the retroviral genome has been described previously. Altering the size of the repeated regions within such a replicated vector is proposed as a means to control its virulence by targeted destabilization of the viral env gene without affecting the expression of the gene of interest. In this regard, this embodiment therefore creates an attenuated replication-competent retrovirus (Sorge et al., J. Mol. Appl. Genet. 1 (1982) 547 ; Hugues et al., Virology 136 (1984) 89).

Alternatively, because deletion of the polynucleotide from the replicating genome may also be observed, the invention also proposes to use any such deletion event as a further means to control viral spread. In particular, by rendering viral replication dependent from the presence of the polynucleotide, it is possible to take advantage of any possible deletion event affecting the polynucleotide. In a more particular embodiment, the replicating viral construct comprises a modified LTR region, which is active in the presence of a polypeptide encoded by (i) the viral construct itself or (ii) another viral construct.

More particularly, a specific object of this invention resides in a replicating viral construct, wherein said viral construct comprises a modified LTR region which is active in the preence of an activating polypeptide, and wherein said viral construct comprises a polynucleotide encoding said activating polypeptide. Even more preferably, the activating polypeptide is encoded by the same polynucleotide that encodes the product whose delivery is sought. The two polypeptides may be produced as a single, fusion molecule, that may be cleaved within the cells upon expression, for instance by digestion of a cleaving site introduced between said two polypeptides. Alternatively, the two polypeptides may be produced as two separate molecules, the respective coding nucleic acids being fused by means of IRES for instance. In these embodiments, any deletion event of the polynucleotide creates a replication-defective viral construct and thus avoids dissemination thereof. In a further preferred embodiment, the activating polypeptide is expressed from the viral construct under the control of a regulated or (tissue or cell) selective promoter, to further control viral replication. Examples of such promoters are described later in this application:

The activating polypeptide may be any transactivator molecule, such as a tetracycline responsive trans-activator or the HIV Tat/tar system, or any other transcription activating molecule, preferably of non-human origin, to ensure higher selectivity of the system. The modified LTR may comprise the operating sequences to which the transactivator binds, thereby activating expression from the LTR. Said operating sequence may for instance be inserted in replacement of all or part of the U3 region of the LTR. The operating sequences may be inserted in one or several tandem copies, for instance between 1-8 copies, more preferably 1 to 5. The tetracycline operator sequences (TetOp) can be prepared by the skilled artisan using conventional techniques. In this regard, another aspect of the present invention also resides in a modified retroviral LTR, wherein said LTR comprises a transcription activator operator sequence in replacement of all or part of the U3 region.

As will be further described below, the activating polypeptide may also be expressed by a separate viral construct. In that situation, not only are the viral constructs dependent from each other with regard to the nature of the proteins produced, but they are also dependent for activating expression thereof.

While insertion of the polynucleotide 3' of the env nucleic acid encoding region as described above represents a preferred embodiment of this invention, it should be understood that other insertion sites or strategies can be used. More particularly, within the replicating viral constructs of the present invention, the polynucleotide may also be inserted:
- 5' of the env gene, the second splice acceptor site being used to regulate env protein expression, or
- 3' of the envelope gene, as a bicistronic unit therewith. More particularly, an IRES sequence (Internal Ribosome Entry Site) can be inserted between the env gene and the polynucleotide, to ensure co-expression of said region from the 5'-LTR,
- 3' of the envelope gene, but in the opposite transcriptional orientation. In this embodiment, transcription of the polynucleotide is controlled by an internal promoter region contained in the polynucleotide which, as described later, may be a regulated and/or cell or tissue selective promoter.
- Although less preferred, the polynucleotide may also be present into other locations of the viral genome, such as for instance in the U3 region of the LTR sequence.

Preferred replicating viral constructs or genomes of this invention, and, in particular, preferred retroviral constructs or genomes of this invention comprising (i) functional gag, pol and env genes, (ii) a polynucleotide to be delivered to cells, (iii) a retroviral packaging sequence and (iv) at least one retroviral LTR sequence, are further characterized in that:
- they encode a modified envelope protein having a modified host range (i.e., retargeted envelope), and/or
- they encode a modified envelope protein comprising a selected epitope, and/or
- they comprise a modified LTR sequence that is active in the presence of an activating polypeptide, and/or
- the polynucleotide encodes αGal4, and/or
- the polynucleotide encodes an immunogenic polypeptide or a cytokine, and/or
- the polynucleotide encodes a (conditionally) toxic molecule, and/or
- the polynucleotide comprises a regulated or selective promoter.

These characteristics can be present within the viral constructs of this invention as any combination thereof. Preferably, at least one or two of said features are present within the constructs.

As indicated above, the replicating (retro)viral constructs of the present invention can be a nucleic acid, a plasmid, a vector or a virus, for instance, comprising the above-disclosed genome.

In one embodiment of the present invention, the retroviral construct is a retroviral vector or plasmid, i.e., a linear or circular nucleic acid (RNA or DNA) comprising a recombinant replication-competent (retro)viral genome as defined above.

Accordingly, in one aspect, the invention resides in a plasmid comprising a recombinant replication-competent retroviral genome as defined above, as well as in any composition comprising such a plasmid. The invention also resides in a method for delivering a polynucleotide to cells, comprising contacting said cells (culture, tissue, organ, etc.) in vitro, ex vivo or in vivo with said plasmid or composition.

This embodiment is advantageous since plasmid or vector constructions, manipulations and production can be performed in any suitable host cell, according to conventional recombinant DNA techniques. For instance, in a particular embodiment of the present invention, the retroviral construct is a plasmid comprising a recombinant, replication-competent retroviral genome as defined above, and an origin of replication functional in a host cell such as a prokaryotic or eukaryotic host cell. The plasmid can therefore be prepared in any convenient host cells such as bacteria (e.g., E.coli) or yeast cells (e.g., saccharomyces, kluyveromyces, etc.), and there is no requirement for a stable packaging cell line, a confined environment, complex purification methods, etc. In a particular aspect of the invention, the plasmid may further comprise a marker gene, further facilitating the construction, manipulation and production thereof in vitro.

Another advantage of this embodiment of the present invention is that high levels of plasmid transduction are not required to obtain efficient gene delivery. Indeed, upon contacting with the cell, culture, tissue, organ, or the like, the plasmid or vector penetrates into the cells and allows the recombinant retroviral genome to be replicated (usually after integration thereof into the host genome). The replicated recombinant retroviral genome is then packaged into retroviral particles, which are formed by assembly of the core and envelope proteins expressed from the functional gag and env genes, and subsequently released outside of the transduced cells. Considering the replication and packaging efficiency of retroviruses, this method allows the production of large amounts of recombinant replication-competent retroviral particles from one plasmid or vector incorporated into one cell. In this regard, the examples which follow clearly demonstrate efficient propagation of the recombinant retrovirus. Accordingly, even where the initial contacting step is not improved or optimized, this method allows the production of large amounts of infectious, recombinant, replication-competent retroviral particles which can spread and infect surrounding cells and allow high polynucleotide transfer efficiency, in vitro, ex vivo or, in vivo.

The invention therefore also resides in methods of delivering a polynucleotide to a cell, in vitro, ex vivo or in vivo, comprising contacting said cell with a plasmid or composition as described above. The invention also resides in the use of a plasmid or composition as described above for the preparation of a composition for delivering a polynucleotide to a cell, in vitro, ex vivo or in vivo.

In a particular embodiment, the contacting step can be performed in the presence of any agent or treatment known to facilitate cell transduction. In this regard, various transfecting agents have been reported in the literature, such as liposomes, cationic lipids, peptides, polymers, etc., as well as physical treatments such as electrical field, gene gun, balistic methods, and the like. Any such treatment and/or method can be applied to the initial contacting step in order to further increase the transduction of the plasmid, if appropriate. Alternatively, the contacting step can be performed with naked DNA plasmid compositions, especially for intramuscular gene delivery. Obviously, any other method, agent, treatment or condition known to improve the contacting step can be used in performing the instant method.

In particular embodiments, the contacting step can be performed by liposome-, cationic lipid-, polymer- or peptide-mediated transfection.

In another particular variant of the invention, a replication-defective virus is used to deliver the above vector/plasmid or, more generally, the replicating viral genome, to the cells. In this regard, the retroviral genome (or vector/plasmid) can be inserted in a defective adenoviral or AAV vector, an herpes amplicon, a vaccinia virus vector or the like. The initial contacting step thus involves the infection of the cell, culture, tissue or organ with the corresponding virus (e.g., adenoviruses, AAVs, HSV, vaccinia) for instance.

According to another embodiment of the present invention, the replicating retroviral construct is a recombinant retrovirus comprising the recombinant, replication-competent retroviral genome as defined above.

Another object of the instant invention therefore resides in a replicating retrovirus comprising a recombinant, replication-competent retroviral genome as defined above, as well as in any composition comprising such a retrovirus, and uses thereof. More preferably, in the replicating genome, the polynucleotide is inserted outside of the LTR sequences, preferably 3' of the env-coding region, even more preferably, downstream of a splice acceptor site.

In another preferred embodiment, the replicating retroviral gene encodes a modified env glycoprotein, e.g., a retargeted env protein with modified tropism, in particular allowing preferential infection of selected cell population(s), tissue(s) or organ(s), and/or an immunogenic env protein (containing a selected epitope) as will be discussed in more detail below.

Obviously, in a particular embodiment, the replicating retroviral genome may combine the above two features, as well as others which will be described later in this application.

If desired, the recombinant retrovirus comprising the recombinant, replication-competent retroviral genome can be produced in vitro, either by transient transfection of a competent cell with the retroviral construct, or from a corresponding stable packaging cell line, i.e., a population of cells comprising, integrated into their genome the replication-competent retroviral genome as defined above.

In vitro production by transient transfection can be accomplished as described above, by contacting a competent cell population with a retroviral vector or plasmid comprising a replication-competent retroviral genome, followed by recovery of the recombinant retroviruses produced. Competent cells can be for instance any mammalian or insect cells, which can be grown in culture, do not exhibit known significant pathogenic activity, and can replicate a retroviral genome. The cell can be established as an immortalized cell line, or a culture of primary cells. More preferably, the competent cell is a mammalian cell, such as a rodent cell, a primate cell or a human cell. Specific examples include, fibroblasts (such as NIH 3T3), retinoblasts, kidney cells (e.g., 293 cells) and the like. Other examples of competent cells or cell lines have been described in EP 243 204, WO 89/07150, WO 90/02806 or WO 93/10218 for instance.

Alternatively, as mentioned above, a packaging cell producing a recombinant replication-competent retrovirus of the present invention can be prepared. For this purpose, a population of competent cells established as a cell line is contacted with a retroviral vector or plasmid comprising a replication-competent retroviral genome and, optionally, a marker gene. Clones of competent cells having stably integrated the retroviral genome can be selected and subcultured. Cell banks can then be prepared, including master cell banks, controlled by various techniques such as PCR for stable integration of the recombinant retroviral genome, and stored under appropriate condition. Within the context of the present invention, stable integration of the genome means that the recombinant retroviral genome remains integrated within the host cell genome over at least 20 generations (i.e., over 20 cell divisions). Production from such packaging cells comprises (i) culturing the cells in appropriate medium and conditions to allow replication of the genome, expression of the core and envelope proteins, packaging and release of the retroviruses, (ii) followed by recovering of the viruses produced.

In this regard, another object of the present invention resides in a retroviral packaging cell, wherein said packaging cell comprises, integrated into its genome, a recombinant replication-competent retroviral genome, as described above. As discussed before, previously described retrovirus packaging cells produce replication-defective retroviruses. In contrast, the packaging cells as presently claimed allow the production of replicating retroviruses.

Where recombinant viruses are being used, the contacting between the population of cells and the recombinant retrovirus may be accomplished in vitro, ex vivo or in vivo by incubating the cells in the presence of a suspension of the retroviruses. The suspension can be a supernatant of a packaging cell culture producing the virus, or a dilution or concentrate thereof. The suspension can also be a partially purified supernatant, enriched for the viruses, obtained according to known methods (i.e., gradient centrifugation, chromatography or the like). The incubation is generally performed with a suspension of retroviruses comprising between 10⁴ and 10⁷, more preferably between 10⁴ and 10⁶ viral particles approximately. It should be understood that the precise amount of viruses per cell used in the method can be adapted by the skilled artisan without undue experimentation. Contacting may also be obtained by cocultivating the target cells, tissue, organs, etc. with packaging cells as described above (for in vitro or ex vivo uses) or by grafting said packaging cells in vivo, as described below.

As illustrated in the examples, the use of (securized) replicating viral constructs according to the present invention provides a very efficient way of delivering nucleic acids to cells, in vitro, ex vivo or in vivo, in particular to proliferating cells, more preferably to tumor cells.

### The polynucleotide

The instant invention can be used to deliver essentially any polynucleotide to a cell, culture, tissue, organ or organism. More preferably, the polynucleotide may be any DNA, cDNA, synthetic or semi-synthetic DNA, RNA, mRNA, etc. The polynucleotide preferably comprises less than 10 kb, more preferably less than 5 kb. Preferred polynucleotides to be used in the instant invention comprise between 200 and 4000 bp. The polynucleotide may additionally comprise a promoter region or other expression signals although, as explained above, the presence of such elements might not be necessary to ensure proper expression.

Preferably, the polynucleotide is heterologous with respect to the retroviral construct, i.e., does not originate from the retrovirus used in the production of the constructs. However, as indicated before, the polynucleotide may also encode viral or retroviral proteins, such as for instance fusogenic retroviral envelope proteins (VSV-G, GALV, etc.) or immunogenic envelope proteins, such as HIV envelope, polio envelope, or modified envelope proteins comprising an antigenic peptide in their sequence. In such embodiments, the polynucleotide would substitute for the env or corresponding retroviral gene. Particular examples include MoMLV retroviral constructs pseudotyped with a heterologous envelope protein-encoding nucleic acid, e.g., a HIV envelope. In such an embodiment, the polynucleotide is the env gene.

Alternatively, the env gene may be modified to contain a sequence encoding an antigenic peptide. In this embodiment, the polynucleotide is therefore a nucleic acid encoding the retroviral, modified, envelope protein.

In another embodiment, the retroviral construct is of the MoMLV type, the envelope is of a different type (e.g., HIV), and the polynucleotide encodes an immuno-stimulating polypeptide, such as a cytokine (e.g., IL-2).

The polynucleotide may encode any product of interest, including one or several RNA, peptide, polypeptide and/or protein. The product encoded may exhibit biological activity, such as therapeutic or immunogenic activities. It may also exhibit toxic activity, a marker property, antisense activity, etc. It is believed that the instant invention can be used by the skilled artisan with essentially any kind of polynucleotide.

The polynucleotide preferably encodes a product which is biologically active in mammalian cells such as human cells.

Examples of polynucleotides include any suicide or toxic gene, in particular conditionally toxic genes such as thymidine kinase, cytosine desaminase, or the like, or other bacterial toxins or fusogenic retroviral envelopes. The polynucleotide may also encode tumor suppressor proteins, such as p53, Rb, E1, BRCA1, etc., cytokines (e.g., IL-2, or any other lymphokines such as TNF, IFN, etc.), antiangiogenic factors, antigenic peptides, growth factors (G-CSF, GM-CSF, BDNF, CNTF, etc.) and the like. The polynucleotide may also encode single chain antibodies, antisense RNAs, Ribozyme and the like.

### Control of the virus spread

As mentioned before, the invention stems from a new concept of using replication-competent viral constructs to deliver genes to cells, in vitro or in vivo. In the prior art, efforts have always been made in order to avoid replication of recombinant viruses (complex packaging cells, highly deleted viral constructs, etc.). The invention now claims that replication of viral constructs can be tolerated, and takes advantage of said replication to increase polynucleotide delivery and expression. It is indeed proposed that replication of some retroviruses (such as MoMLV retroviruses) would not induce significant pathological condition in subjects. In particular, it is proposed that such potential adverse effects (i) are strongly balanced by the biological benefits obtained by this delivery system and (ii) can be controlled by several methods and constructs (for instance where other retroviruses are being used). For this purpose, several strategies are possible, which represent preferred, specific embodiments of the instant invention. These strategies can be used alone or in combinations.

As a preliminary remark, it should be noted that certain retroviruses, such as MoMLV essentially infect dividing cells, so that the spread of the retroviral genomes constructed with MoMLV sequences is limited to such dividing cells and would not normally extend to quiescent cells. However, even where other retroviruses are used, such as lentiviruses, which infect also quiescent cells, virus spread may be controlled or limited using any one of the following methods or constructs, either alone or in combination(s).

In one particular embodiment, the virus spread is further controlled by retargeting the viruses. Indeed, the tropism of a retrovirus is determined essentially by the envelope glycoprotein. The retroviral surface protein SU of the envelope is mainly responsible for the binding of the virus to a specific cell surface receptor, and the TM subunit triggers post-binding events, leading to membrane fusion and viral entry (Ragheb et al., J. Virol. 68 (1994) 3207). It has been disclosed that the envelope glycoprotein can be modified, for instance to incorporate specific receptor ligand or receptor fragment, and that the resulting envelope proteins provide targeted infection of cells expressing said receptor or ligand. Accordingly, in order to control spread of the viruses, in a preferred embodiment of the invention, the envelope protein encoded by the replicating or semi-replicating viral genomes has a modified tropism that retargets the specificity of retroviral attachment. Examples of such envelope proteins with modified tropism include membrane proteins with affinity for CD4, retroviral envelope proteins comprising a TM subunit fused to a peptide, such as a Hepatocyte Gowth Factor fragment (Nguyen et al., Hum. Gene Ther. 9 (1998) 2469), as well as amphotropic envelope proteins fused, at their N-terminal end, with ligands such as single chain antibodies, for instance.

Virus spread can also be controlled by the use of tissue specific and/or regulated (e.g., inducible) promoters, i.e., promoter regions which are particularly active in preferred cells, tissues or organs and/or under certain conditions. In this regard, targeted expression control can be introduced at various levels, depending on the structure of the viral constructs which are being used. For instance, where the polynucleotide comprises its own promoter region, targeted expression of the polynucleotide can be accomplished by introducing a selective and/or regulated promoter in said polynucleotide. Alternatively, where expression of the polynucleotide is controlled by the promoter region of the LTR, it is possible to modify the LTR region in order to confer selective and/or regulated expression thereto. In this respect, the 3' U3 region of the LTR can be engineered with any tissue selective/regulated regulatory sequence, so that, after reverse transcription, the 5' LTR controls viral genes and polynucleotide expression with said regulatory sequences.

Several candidate selective and/or regulated promoter or regulatory regions can be used, such as promoter (or fragments thereof) allowing preferential and/or regulated expression in certain tumor cells (e.g., hepatocarcinoma, colon carcinoma, glioblastoma) or other abnormally proliferating cells, including for instance cells of the hematopoietic or nervous system. Particular examples of selective and/or regulated promoters include for instance:
- the alfa-fetoprotein (AFP) promoter, which is expressed in about 40% of primary liver tumors (Mawatari et al., Cancer Gene Ther. 5 (1998) 301). Previous studies have shown that human AFP promoter allows preferential expression of a reporter gene in hepatocarcinoma cells. Furthermore, selective hepatocarcinoma expression with AFP promoter has been observed in the context of retroviral vectors.
- the Aldolase A promoter, which is expressed in liver tumors, particularly in patients with HCC (Moch et al., Transgenic Research 7 (1998) 113 ; Guillouzo et al., J. Cell Sci. 49 (1981) 249).
- the Pancreatic-associated protein (PAP/HIP) promoter, which is overexpressed in up to 70% of hepatocarcinoma (Christa et al., J. Hepatol. 30 (1999) 105). The PAP/HIP promoter appears to be tightly regulated in HCC cells, and a 1.3 kb fragment thereof directs highly preferential expression into HCC cells.
- the carcinoembryonic antigen (CEA) promoter, which is particularly active in colon carcinoma (CC). This promoter has already been shown to remain selective in a viral context (Richards et al., Hum. Gen. Ther. 6 (1995) 881).
- the glial fibrillary acidic protein (GFAP) promoter and the myelin basic protein (MBP) promoter, which have both been shown to preferentially initiate transcription in glioblastoma cells (McKie et al., Gene Ther. 5 (1998) 440 ; Morelli et al., J. Gen. Virol. 80 (1999) 571).
- interleukin promoters, which may be used to provide preferential expression in certain hematopoietic cells,
- any promoter, including tissue-selective promoters (such as neural and endocrine promoters with neuronal, glial and pituitary specific activity for instance) combined with regulatory sequences, such as the tetracycline-regulatable expression sequences (Smith-Arica et al., Cold Spring Harbor, Spring Meeting, 1999) or any other modified transcriptional activator.

It should be understood that any other promoter or regulatory sequence conferring selective and/or regulated expression may be used by the skilled artisan.

In another particular embodiment, the virus spread is controlled by the product encoded by the polynucleotide itself. For instance, the polynucleotide may comprise a sequence encoding a (conditionally) toxic molecule, that can be used to destroy infected cells. Such a conditionally toxic molecule can be for instance a thymidine kinase, cytosine desaminase, derivatives thereof and the like, which are able to convert a metabolite (e.g., a nucleoside analog or 5-FU, respectively) into a product which destroys the infected cells. The sequence encoding a (conditionally) toxic molecule may be added into the polynucleotide, in combination with another coding sequence producing a biological product. It may of course be used also as a biologically active gene/product, for instance for cancer treatment. The polynucleotide may also encode other toxic molecules such as fusogenic envelope proteins, leading to infected cell death.

In another particular embodiment, the replicating viral construct encodes a polypeptide that renders infected cells sensitive to immune cells and thus causes or stimulates their elimination by the host organism. A particular example of such a polypeptide is α-galactosyl transferase (αGAL4), as described in Gollogly et al (Neoplasma 43 (1996) 285). αGAL4 is a bacterial enzyme not expressed in human cells, which creates particular glycosylation motifs. More particularly, αGAL4 introduces alpha(1-3) galactosyl motif on glycolipids, which motif is not present on human cells. This motif is however expressed by many bacteriae, and human beings have high levels of natural antibodies directed against it. By introducing a nucleic acid encoding such a polypeptide in the replicating viruses of the present invention, the viral particles can be neutralized and infected cells destroyed by the host's immune system, thereby controlling viral spread. Furthermore, the αGAL4 may also act as the therapeutic gene or product, where destruction of diseased cells (such as tumor cells) is sought. In this regard, in a particular embodiment, the present invention describes in a replicating viral constructs comprising a nucleic acid encoding a αGAL4 polypeptide.

Other examples of such polypeptides include antigenic peptides, more preferably from microorganism against which people are or can be vaccinated (such as antigenic peptide from tetanus toxoid or from hepatitis) and/or immuno-stimulating molecules (such as interleukin-2 for instance or other cytokines). Expression of such polynucleotide raises an immune response of the host against said peptides, proteins and/or molecules, leading to an immune reaction against cells infected with the retroviral constructs. This immune response therefore (i) produces the expected biological effect of polynucleotide delivery and expression and (ii) leads to an elimination of infected cells in the organism. Accordingly, the use of the instant invention to raise an immune response (i.e., cellular or antibody) is advantageous since it has the effect of controlling virus spread when the desired immune effect is obtained.

In this regard, a particular embodiment of this invention resides in a composition of replicating viral constructs as defined above, wherein said viral constructs comprise, in combination or separately, a polynucleotide encoding αGAL4 or an antigenic peptide and a polynucleotide encoding a cytokine, more preferably IL-2. More preferably, the composition comprises at least a first replicating viral construct comprising a polynucleotide encoding αGAL4 or an antigenic peptide, and at least a second second replicating viral construct comprising a polynucleotide encoding a cytokine, more preferably IL-2, for simultaneous, separate or sequential use.

Still another approach to control virus spread in vivo, comprises the administration of neutralizing compounds, such as neutralizing antibodies or antiviral compounds, which can inactivate the retroviruses. In this regard, several retrovirus-neutralizing monoclonal antibodies have been disclosed in the prior art. When the vector comprise an antigenic peptide from a micro organism such as tetanus toxoid, immune sera raised against this microorganism that are commonly used in the clinic can be used advantageously in this setting. Suitable anti-viral compounds also include, for instance, AZT (zidovudine) or other didesoxynucleotide analogs such as DDC (didesoxycytosine) and DDI (didesoxyionisine), antiproteases, such as protease p12 and protease p15 inhibitors, combinations thereof, vaccines and the like. In a particular embodiment, the method of delivering polynucleotides in vivo of the present invention therefore comprises the co-administration of (i) replicating retroviral constructs as disclosed above and (ii) a neutralizing compound, such as a neutralizing monoclonal antibody (or derivatives thereof such as Fab fragments, single chain antibodies, etc.) or an anti-viral compound or treatment. In a particular embodiment, the replicating retroviral constructs are administered in a tissue or organ and the neutralizing compound is administered by intravenous injection, for instance. Co-administration does not imply simultaneous administration, but indicates that, in order to provide control over virus spread, the neutralizing molecules should be administered (i.e., either prior to the retroviral constructs, or at about the same time, or even subsequently).

Moreover, control of virus spread may also be achieved advantageously by using retroviral constructs with (modified) envelope proteins against which the host organism is already immunized, or can be easily immunized. In this regard, where the host already exhibits anti-env antibodies, viral spread will be limited. Alternatively, in order to control virus spread, the host subject may be immunized, prior to or at about the same time, or after administration of the retroviral construct (e.g., by using a polio vaccine where the retroviral construct is pseudotyped (expresses) the polio envelope, optionally in combination with other retroviral envelopes, such as MoMLV or HIV envelopes). Alternatively, the envelope protein may be modified as described above, to contain an epitope that is recognized by antibodies or immune cells of the host organism. In this regard, epitopes may be introduced in the envelope protein without significantly altering the activity of the envelope, said epitopes being exposed at the surface of the viral particle or at the surface of cells infected therewith. The epitope may be selected among epitopes against which human beings are or can be easily immunized. Such epitopes include for instance any immunogenic peptide derived from tetanus toxin, flu virus, poliovirus, measle virus, hepatitis viruses... The epitope or peptide, should be of sufficient size to ensure a proper conformation allowing recognition by antibodies or could be short sequences generally below 20 amino acids, more preferably below 15 amino acids, when recognition by CTLs is sought. The antigenic peptide is preferably introduced at the C-terminal end of the envelope, to ensure exposure thereof. Upon administration (or in vivo production) of such viruses, the viral particles that disseminate can be neutralized and eliminated by antibodies of the host and the infected cells destroyed by antibodies or immune cells. In this respect, in a particular embodiment, the epitope(s) comprises at least a class I CMH epitope, that is recognized by CTL lymphocytes, leading to elimination of infected cells. These represent particular embodiments and methods / or controlling viral spread.

### Envelope modification to increase packaging efficiency

As mentioned above, the inventors of the present invention have surprisingly shown that the env gene exerts a negative effect on encapsidation of a retroviral genome in a retroviral particle. This unexpected discovery opens new strategies to increase retroviral vectors' packaging efficiency by modifying the env gene. In this regard, the invention also describes in a method of increasing packaging efficiency of a retroviral vector, the method comprising modifying the env gene in said retroviral vector to create a non-functional env gene. Modification more preferably comprises deleting all or part of the env gene, more preferably the entire env coding sequences. This method can be applied to all types of retroviral vectors as mentioned above, and is particularly suited for producing inter-dependent retroviral vector systems in which at least two retroviral constructs are being used (either simultaneously or sequentially), one of which being env-defective.

As will be disclosed in the following examples, the instant invention now provides very efficient compositions and methods for delivering polynucleotides to cells in vitro, in vivo or ex vivo. The invention can be used for experimental research, clinical research, in human and/or animal, as well as for therapeutic, diagnostic or prophylactic treatments.

### LEGEND TO THE FIGURES

Figure 1: Construction of plasmid pGH45
Figure 2: Construction of plasmid pGH2
Figure 3: Molecular design of the replication competent MoMLV vector. In MoMLV, the open reading frames gag-pol and env are expressed from a common promoter in the 5'-LTR (arrow) by alternative splicing. To construct the replication competent vector GH20, an additional splice acceptor site was introduced downstream of the env gene of MoMLV by duplicating 0,4kb of the pol sequence containing the natural acceptor region (SA). The reporter gene EGFP was placed downstream of the second SA. The spacing between the SA site and the start codon of the transgene is similar to that of the env transcription unit.
Figure 4: Propagation of the vector. Subconfluent monolayers of NIH 3T3 cells were infected with the replicative construct GH20 (A) or with a non replicating EGFP expressing vector (B) at a low m.o.i. Fluorescent focuses were photographed 3 days post infection.
Figure 5: Serial infections. NIH 3T3 cells were infected with the vector GH20 at maximal 10³ focus forming units per well. After 3 days supernatants were harvested and diluted for the next round of infection. The supernatants were tittered on NIH 3T3 cells by fluorescence microscopy.
Figure 6 : Titration of the vector. 500µl of supernatant from a GH20 producer cell pool was used to infect 10⁵ NIH 3T3 cells. Three days post infection, the titer was estimated by fluorescence microscopy. The titer was compared to the percentage of EGFP expressing cells as determined by FACS (A). In order to disclose the settings for the FACS analysis, the infection with undiluted supernatant (B) and uninfected NIH 3T3 cells (C) are shown.
Figure 7 : PCR assessment of wt rearrangements.
   A) A vector specific PCR product was amplified spanning from the MoMLV env to the 5' -terminus of the EGFP reporter gene.
   B) A primer pair binding to the env gene and to the U3 region of the LTR, respectively, gives a 1 kb fragment with MoMLV wt DNA as template. A vector specific fragment of the theoretic size of 2kb is not amplified under the chosen parameters. Lysates of 5x10⁴ cells per reaction were analyzed, control reactions (lanes 1-3) were performed in an equivalent background of NIH 3T3 genomic DNA.
Figure 11: Sequence SEQ ID NO:6. Nucleotide position 1 corresponds to nucleotide 7801 in pGH45. Element (I) at nt 427 is the stop codon of env. Element (II) at nt 437 represents the beginning of repeated pol region containing the SA site, which ends at element (IV), at nt 802, This region 437-802 is a particular fragment comprising a splice acceptor site. Element (III), nt 532 - nt 540 represent the splice acceptor site. Element (V) at nt 840 (ATG) is the start codon of EGFP gene.
Figure 12: Comparison of transgene expression and therapeutic gene transfer efficacy using replicative or defective retroviral vectors transducing a TK/GFP fusion protein. Bars represents the mean tumor weight for tumors transduced with a replicative or defective vector, and treated or not with ganciclovir. For each group, the proportion of GFP expressing cells is indicated by the dashed area of the bar.
Figure 13: Treatment of established tumor by replicative or defective vectors transducing a TK/GFP fusion gene.

### EXAMPLES

### A - Preparation and uses of replicating viral constructs

In order to construct the replicating MoMLV vector pGH45, the reporter gene was placed downstream of the env gene, preceded by a copy of the viral splice acceptor region. The constructs described are of the genomic structure LTR-gagpol-env-EGFP-LTR. Between the env gene and the EGFP reporter gene a 0,4kb fragment of the MoMLV pol gene, containing the known critical regions of the splice acceptor, were inserted. The mechanisms of splice control in MoMLV are not well understood so far. While expression levels of the transgene depend on the acceptance of the additional splice acceptor, over-splicing at this site is expected to abolish virus growth by reduction of the amount of full length genomic RNA, or by interference with the processing of the env message. Surprisingly, the chosen configuration allows both efficient expression of the reporter gene and replication of the viral vector.

Furthermore, the intended attenuation of the vector by targeted deletion of the env gene in GH45 was observed at high frequency. Clones of infected cells were tested for the expected deletion by PCR (data not shown). About 30% (8 from 28) of the EGFP expressing clones, isolated after three rounds of infection, displayed the env deleted structure in PCR testing.

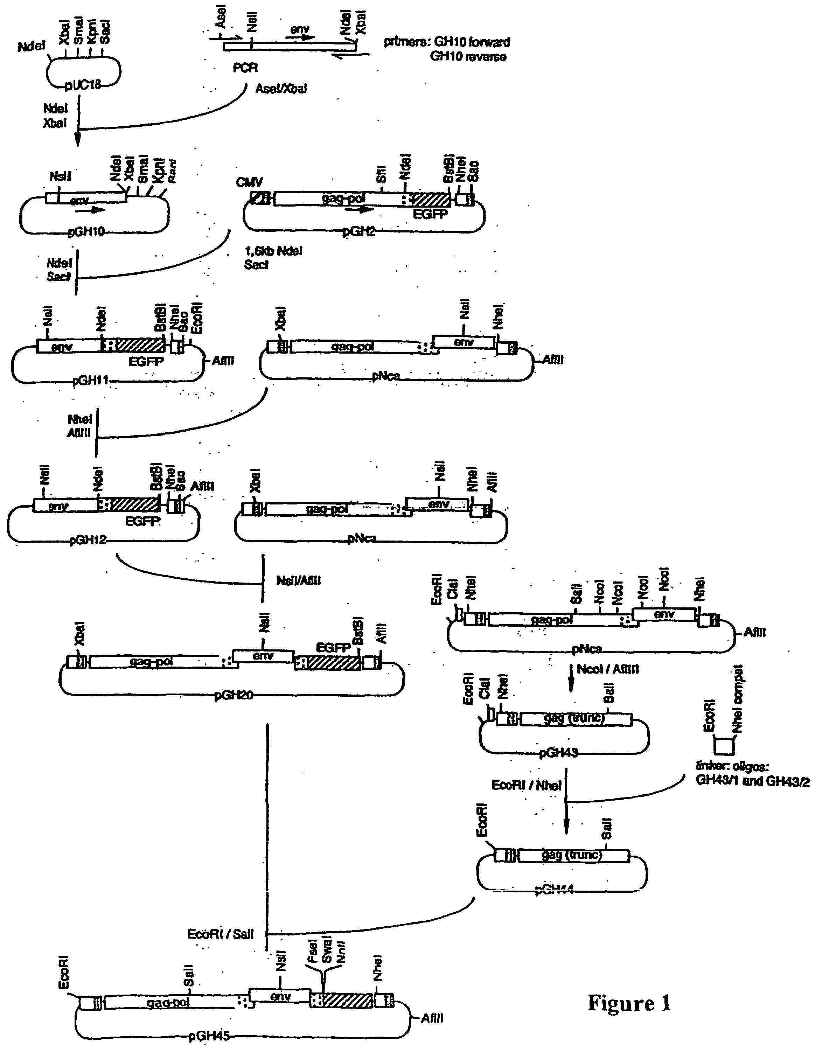

### A.1. Construction of the replicative vector pGH45 (FIGURES 1-3)

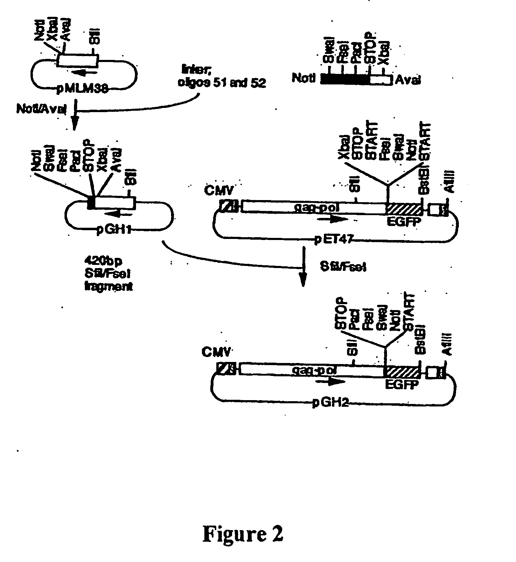

The plasmids pMLM38 et pET47 were used as a source for the sequences around the splice acceptor site to be introduced into the vector. The transcriptional start site of the transgene at the intended splice acceptor site contained an additional aberrant ATG signal and was corrected as follows:
pGH1
   The plasmid pMLM38 was cut with Notl and Aval and a synthetic linker consisting of the oligonucleotides GH51 (5'-GGCCGCTA TTTAAATGGC CGGCCTTAAT TAAAGTCTAG AGGATGGTCC ACCC ; SEQ ID NO: 2) and GH52 (5'-CCGGGGGTG GACCATCCTC TAGACTTTAA TTAAGGCCGG CCATTTAAAT AGC ; SEQ ID NO: 3) was inserted. The resulting plasmid was termed pGH1.
pGH2 (Figure 2)
   A 0,4kb fragment containing the corrected region was isolated from pGH1 by Sfil and Fsel digestion and cloned into Sfil / Fsel cut pET47. The resulting plasmid pGH2 is identical to pET47 with exception of the improved transcriptional start region of the transgene.

The assembly of the replicative vector pGH2, containing the entire MoMLV proviral genome, including the open reading frames gap-pol and env and the EGFP reporter gene was done as follows:
pGH10
   The 3' prime portion of the MoMLV env gene was amplified as a 0,75 kb PCR fragment with the primers GH10 forward (AGTACCGGGA TTAATCCATG CATCTCCACC ACCATACTG ; SEQ ID NO: 4) and GH10 reverse (TATGGTCTCT AGACATATGC TAT GGCTCGT ACTCTATAGG CTTCAGC ; SEQ ID NO: 5) and the plasmid pNca as template. The PCR product was cut with the enzymes Asel and Xbal and ligated int Ndel / Xbal cut pUC 18 to give pGH10.
pGH11
   The plasmid pGH2 was digested with Ndel and Sacl to isolate a 1,6kb fragment containing 400bp of the 3' -terminal of the MoMLV pol gene, the entire EGFP reading frame and 3' -LTR sequences. Ligating this fragment to Ndel / Sacl cut pGH10 resulted in the plasmid pGH11.
pGH12
   pGH11 was cleaved with Nhel and AflIII to insert a 0,75kb fragment from pNca (Colicelli J., J. Mol. Biol. 199 (1988) 47-59) thereby completing the 3' -LTR. The resulting plasmid was termed pGH12.
PGH20
   A bp fragment was obtained by digestion of pGH12 by the enzymes Nsil and AflIII. This fragment was ligated into Nsil / AflIII cut pNca to give pGH20. The plasmid pGH20 contains a functional replicating EGFP expressing retroviral genome.

The elements of the vector pGH20 are mapped as follows (see Figures 1 and 3):
1 - 593 5' -LTR
1070 - 2684 gag, complete coding sequence
1070 - 6284 gag-pol, complete coding sequence
5942 - 5951 splice acceptor site
6226 - 8223 env ecotropic, complete coding sequence
8231 - 8596 pol sequences, partial (NdeI-XbaI)
8316 - 8325 splice acceptor site (SEQ ID NO: 6)
8634 - 9353 EGFP gene, complete coding sequence
9416 - 10008 3' -LTR

The vector pGH20 was improved by deleting plasmid sequences that lead to homologous recombination and thus to occasional wt-formation during replication. The improved construct designated pGH45 was constructed as follows (see Figure 1):
pGH43
   In order to manipulate the region upstream of the 5'-LTR, the 3'-half of the retroviral sequences were deleted from the plasmid pNca by digestion with the enzymes Ncol and Afllll and religation. The resulting plasmid was termed pGH43.
pGH44
   pGH43 was cleaved with EcoRI and Nhel and a synthetic linker consisting in the annealed oligonucleotides GH43/1 (AATTCAATGA AAGACCCCAC CTGTAGGTTT GGCAAC ; SEQ ID NO: 7) and GH43/2 (CTAGGTTGCC AAACCTACAG GTGGGGTCTT TCATTG ; SEQ ID NO: 8) was inserted. Thus, in the resulting plasmid pGH44, 160bp of the plasmid sequence upstream of the proviral genome are removed.
pGH45
   A 4,2 kb fragment containing the corrected sequence was isolated from pGH44 by EcoRl / SalI digestion and ligated into EcoRI / SalI cut vector pGH20. The resulting corrected plasmid is termed pGH45 and is of the same proviral sequence as pGH20.

Structural map of pGH45
7 - 599 5'-LTR
1076 - 2692 gag
1076 -6292 gag-pol
5948-5957 splice acceptor site
6232-8229 env ecotropic
8237-8600 pol sequences (NdeI/XbaI)
8322-8331 splice acceptor site
8640-9359 EGFP gene
9422-10014 3'-LTR

The construction scheme for and structure of pGH45 are depicted in Figures 1-3.

The viral proviral sequence from the beginning of the 5'-LTR to the 3'-end of env in the plasmid pGH45 corresponds to the wt-sequence as present in pNca. Downstream of the env gene, the sequence contains the 3' -terminal part of the pol gene, the EGFP gene and the 3'-LTR as present in the plasmid pMLM38. The repeated pol sequence containing the splice acceptor site consists in a 0,4 kb fragment spanning from the Ndel site to the Xbal site present in the MoMLV pol gene, comprising SEQ ID NO: 6 or a variant thereof.

### A2. Propagation and Transgene expression

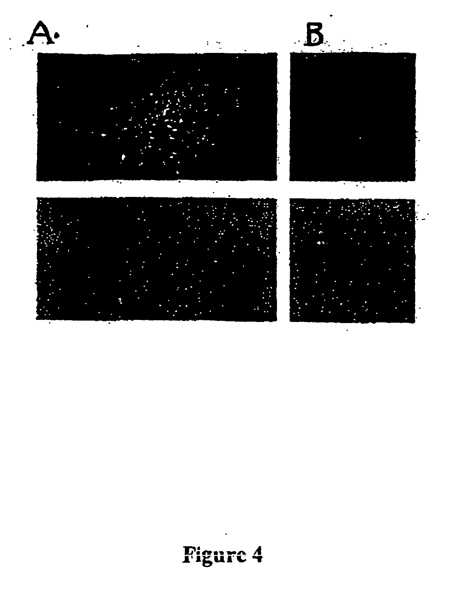

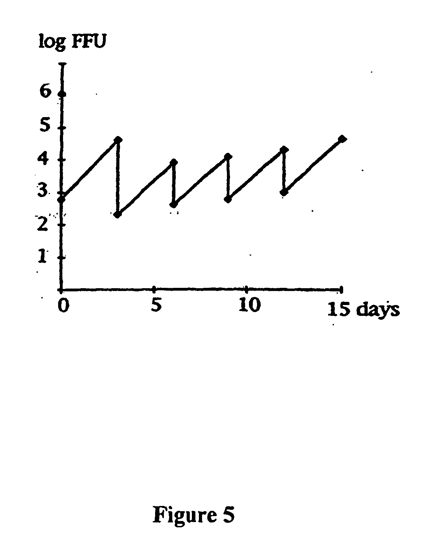

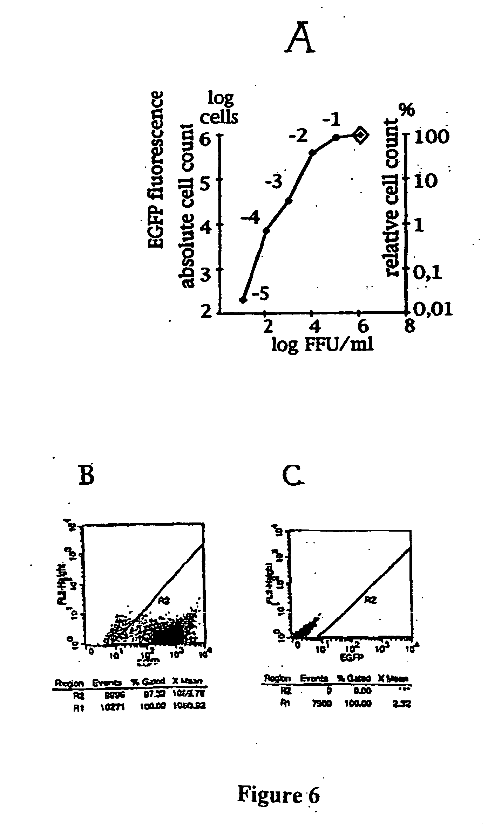

Proviral DNA was transfected into NIH 3T3 cells by calcium phosphate precipitation, and supernatants were drawn 3 days post transfection. Infected NIH 3T3 cells were used for the establishment of producer pools and for repeated infection experiments. The vector displayed substantial EGFP expression and replication as demonstrated by fluorescent focus formation following infection in vitro (Figure 4) and by induction of syncytia on RatXC cells. The growth properties were assessed in 5 repeated infections on NIH 3T3 cells. Over this 15 days period, propagation remained relatively stable at a level of one to two logs of FFU (Fluorescent focus forming units) per 3 days infection (Figure 5). The functionality of the construct is an indication of balanced expression of the reporter and the viral proteins. The titer of producer pools as determined by fluorescence microscopy and by FACS analysis (Figure 6) is about 10⁶ FFU per ml. In infections at low m.o.i., one FFU corresponds to 10² EGFP expressing cells, reflecting the propagation of the vector.

### A3 Genetic Stability

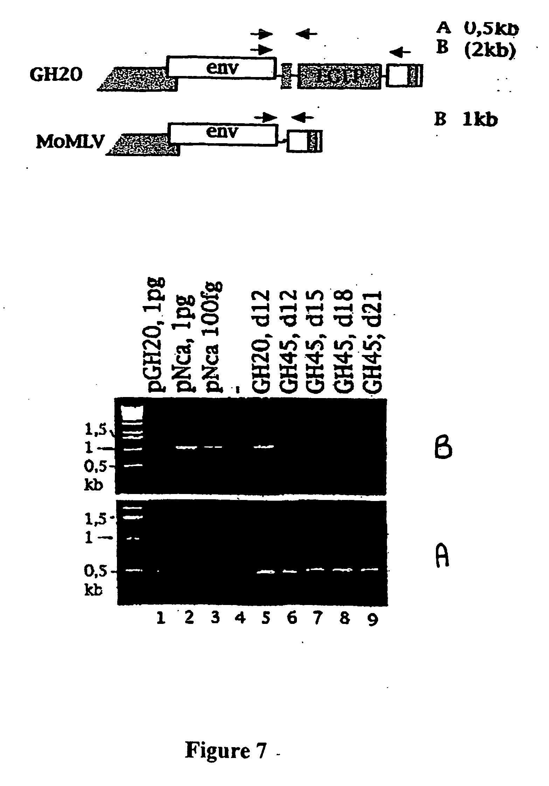

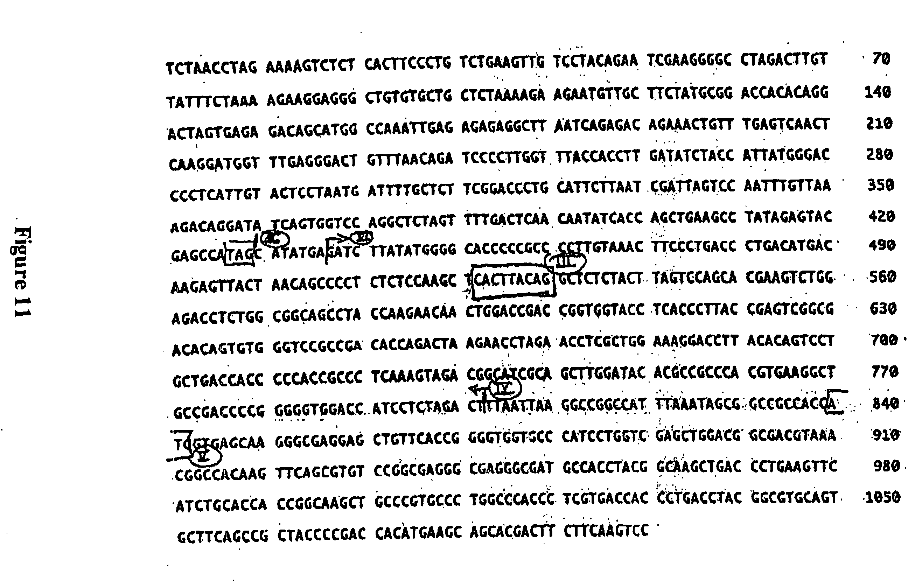

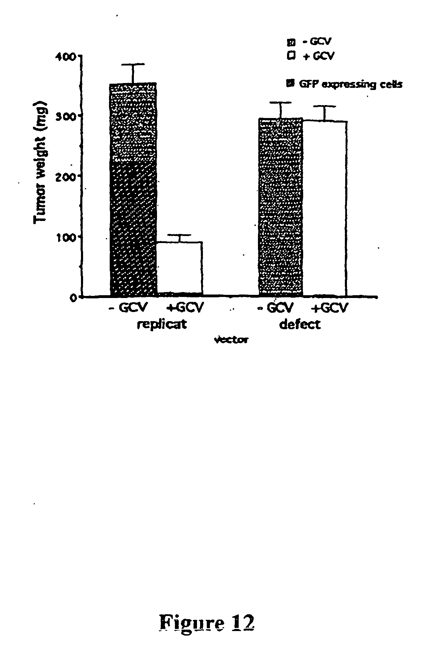

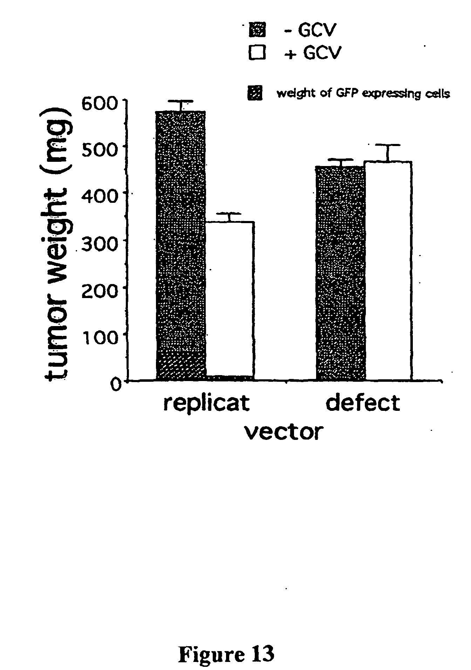

Genomic rearrangements leading to wild type (wt) formation are a well known problem with retroviral vectors in general. Especially, with replication competent constructs, loss of transgene expression due to deletions has frequently been observed. The genetic integrity of the vector was therefore followed by PCR. Lysates of the infected cells were subjected to PCR reactions specific for wt arrangements or for the original vector structure, respectively. The detection limit of the assays was below 5x10³ copies per reaction. The design of the PCR is depicted in Figure 7. A first construct, designated GH20, displayed wt-reversion, being detectable yet after three passages. With an improved version of the vector (GH45), after six passages (d21), no wt specific band was detectable in the PCR assay, indicating a low reversion rate. Interestingly, the genomic sequence of the initial construct GH20 is identical to that of GH45, but 150bp of retroviral sequences are present outside the genomic transcription unit in the GH20 plasmid DNA, directly upstream of the 5'-LTR. As this sequence is excluded from viral replication, homologous DNA recombination during transfection most probably accounts for the observed rearrangements.

### A4. Arrestment of the vector by targeted deletion

The duplication of the SA region in the vector resulted in a 0,4kb tandem repeat, flanking the env gene (Figure 3). Deletion of genomic sequences due to repeated sequences in the retroviral genome and its application for targeted rearrangements has been described previously. Indeed, in GH20, the loss of the env gene by recombination between the repeats was observed at high frequency. Clones of infected cells were tested for the expected deletion by PCR (data not shown). About 30% (8 out of 28) of the EGFP expressing clones, isolated after three rounds of infection, displayed the env deleted structure in PCR testing. Altering the size of the repeated regions in the genome should provide a means to control the virulence of the vector by targeted destabilization of the viral env gene without negatively affecting the expression of the gene of interest.

### DISCUSSION

Retroviral vectors, that are stably expressing a transgene and that are replication competent in mammalians represent -due to a notably enhanced transduction efficiency- a useful tool for delivering polynucleotides to cells, in particular for suicide gene therapy. We attempted a multiple alternative splice approach for the expression of a reporter gene in MoMLV. The efficient translation of the transgene and the propagation of the vector strongly indicate a balanced distribution of the splice products. Of course, variants of the presented vector with alterations in the second splice acceptor region can be constructed in order to improve the effects on splicing and on the arrestment rate by env deletion. Following the genomic stability of the construct by PCR revealed no revertant structures arising during a period of 21 days.

### C. In vivo tumor regression using replicating or semi-replicating viral constructs

This example confirms the efficacy of the present invention, by illustrating tumor regression in vivo using replicating viral constructs expressing a thymidine kinase polypeptide. More particularly, packaging cells producing replicative retroviral construct were injected into mice, either in combination with tumor cells or directly into established tumors. Gene transfer was assessed as well as tumor regression (or size) upon administration of a nucleoside analog (e.g., gancyclovir).

Two packaging cell lines were used in these experiments:
- classical packaging cells (control) expressing non packageable gag/pol and env genes together with a packageable defective retroviral vector, and thus producing infectious but defective retroviral particles,
- cells expressing both a packageable replicative wild type retrovirus and a packageable defective retroviral vector (i.e., two transcomplementing retroviral constructs), and thus producing infectious and replicative particles.

### C1. Co-injection of tumor cells and packaging cells

In order to compare the properties of replicative and defective retroviral vectors in a quantitative manner, mixture of DHDK12 tumor cells (98%) and packaging cell lines (2%) producing either replicative or defective retroviral vectors transducing TK/GFP were prepared. 10 millions cells were injected subcutaneously in nude mice (day 0). At day 7, animals were separated in two groups, one receiving ganciclovir (150 mg/kg/jour) for 7 days. Mice were then sacrificed (day 14). The tumors were dissected, wheighted, and the percentage of GFP expressing cells was measured after dilaceration by flow cytometry analysis.

The results of this experiment are presented on Figure 12. These results demonstrate that (i) gene transfer is much more efficient with a replicative than a defective vector as assessed by the proportion of GFP expressing cell in the groups not receiving ganciclovir; (ii) GCV treatment reduces dramatically the number of GFP expressing cells in both groups; (iii) a significant reduction of the tumor volume is only observed in the group receiving the replicative vector and treated with ganciclovir in accordance with the efficiency of transgene transduction. It should be noted that this experiment was performed in nude mice and therefore there is not a full eradication of the tumor which requires a competent immune system.

### C2. Injection of packaging cells into established tumors

Subcutaneous tumors were first generated in nude mice by injection of DHDK12 cells. After 7 days, when the tumors are palpable, they were injected with approximately 3x10⁶ packaging cells releasing either a replicative or a defective retroviral vector transducing TK/GFP. 7 days later, animals were treated or not with ganciclovir for 7 days. Tumor were analysed as for fig.12.

The results presented on Figure 13 show that only replicative vectors lead to a significant transduction of the tumor cells and therapeutic effect.

## Claims

1. The use of a replicating retroviral construct, said replicating viral construct comprising a replicating retroviral genome for the preparation of a pharmaceutical composition for gene delivery into cells in vivo, ex vivo or in vitro for prophylactic or therapeutic treatment of a mammalian subject, said replicating viral construct comprising a replicating retroviral genome comprising in the 5' -> 3' order:
- a retroviral 5'-LTR,
- a retroviral packaging sequence,
- functional gag, pol and env genes,
- a polynucleotide, and
- a retroviral 3'-LTR,
the retroviral genome comprising a splice acceptor site between said env gene and said polynucleotide..

2. The use of claim 1, wherein said replicating retroviral genome encodes a retroviral envelope having tropism for a mammalian cell.

3. The use of claim 2, wherein said genome encodes a modified envelope glycoprotein.

4. The use of any one of claims 1 to 3, wherein the polynucleotide is under the transcriptional control of a regulated promoter.

5. The use of claim 3, wherein the modified envelope glycoprotein comprises a selected epitope.

6. The use of claim 3, wherein the modified envelope glycoprotein has a modified host range.

7. The use of any one of claims 1 to 6, wherein said viral construct comprises a modified LTR region which is active in the presence of an activating polypeptide, and wherein said viral construct comprises a polynucleotide encoding said activating polypeptide.

8. The use of any one of claims 1 to 6, wherein said construct comprises a polynucleotide encoding αGAL4.

9. The use of any one of claims 1 to 8, wherein said viral construct is a plasmid or a defective adenoviral vector.

10. A pharmaceutical composition comprising a replicating retroviral construct or genome, wherein said replicating retroviral construct or genome comprises in the 5' -> 3' order:
- a retroviral 5'-LTR,
- a retroviral packaging sequence,
- functional gag, pol and env genes,
- a polynucleotide, and
- a retroviral 3'-LTR,
the retroviral genome comprising a splice acceptor site between said env gene and said polynucleotide and the encoded retroviral envelope having tropism for a mammalian cell.

11. The pharmaceutical composition of claim 10, wherein said genome encodes a modified envelope glycoprotein.

12. The pharmaceutical composition of any one of claims 10 to 11, wherein the polynucleotide is under the transcriptional control of a regulated promoter.

13. The pharmaceutical composition of claim 11, wherein the modified envelope glycoprotein comprises a selected epitope.

14. The pharmaceutical composition of claim 11, wherein the modified envelope glycoprotein has a modified host range.

15. The pharmaceutical composition of any one of claims 10 to 14, wherein said viral construct comprises a modified LTR region which is active in the presence of an activating polypeptide, and wherein said viral construct comprises a polynucleotide encoding said activating polypeptide.

16. The pharmaceutical composition of any one of claims 10 to 14, wherein said construct comprises a polynucleotide encoding αGAL4.

17. A recombinant, replicating retroviral genome comprising a polynucleotide inserted outside of the LTR sequence and encoding a retroviral envelope having tropism for a mammalian cell, wherein said viral construct comprises a modified LTR region which is active in the presence of an activating polypeptide, and wherein said viral construct comprises a polynucleotide encoding said activating polypeptide.

18. A recombinant, replicating retroviral genome comprising a polynucleotide inserted outside of the LTR sequence and encoding a retroviral envelope having tropism for a mammalian cell, wherein said construct comprises a polynucleotide encoding αGAL4.

19. A recombinant replicating retroviral genome of claim 17 or 18, wherein the polynucleotide is inserted downstream of the env gene and wherein a splice acceptor site is inserted between said env gene and said polynucleotide.

20. A replicating retrovirus, wherein said replicating retrovirus comprises a retroviral genome or construct according to any of claims 17 to 19.

21. A plasmid, wherein said plasmid comprises a replicating retroviral genome, in particular a replicating retroviral genome or construct according to any of claims 17 to 19.

22. A composition comprising a retrovirus of claim 20 or a plasmid of claim 21.

23. A method for delivering a polynucleotide to a cell, tissue or organ, in vitro or ex vivo, comprising contacting said cell, tissue or organ with a composition of claim 22 or with a retrovirus packaging cell, comprising, integrated into its genome, a replicating recombinant retroviral genome according to any of claims 17 to 19.

24. Use of a composition of claim 22 or a retrovirus packaging cell, comprising, integrated into its genome, a replicating recombinant retroviral genome according to any of claims 17 to 19 for the preparation of a composition for delivering a polynucleotide to a cell, tissue or organ, in vivo, ex vivo or in vitro.

25. Use of a replicating retroviral construct for the preparation of a pharmaceutical composition for gene delivery into cells in vivo, ex vivo or in vitro for prophylactic or therapeutic treatment of a mammalian subject(s), said replicating viral construct comprising a replicating retroviral genome comprising a polynucleotide inserted outside of the LTR sequence and, in the 5' -> 3' order,:
- a retroviral 5'-LTR,
- a retroviral packaging sequence,
- functional gag, pol and env genes, and
- a retroviral 3'-LTR,
said retroviral construct being a plasmid or a defective adenoviral vector.

26. Use of a replicating retroviral construct for the preparation of a pharmaceutical composition for gene delivery into cells in vivo, ex vivo or in vitro for prophylactic or therapeutic treatment of a mammalian subject(s), said replicating viral construct comprising a replicating retroviral genome comprising a polynucleotide inserted outside of the LTR sequence and, in the 5' -> 3' order,:
- a retroviral 5'-LTR,
- a retroviral packaging sequence,
- functional gag, pol and env genes, and
- a retroviral 3'-LTR,
said viral construct comprising a modified LTR region which is active in the presence of an activating polypeptide, and said viral construct comprising a polynucleotide encoding said activating polypeptide.

## Patentansprüche

1. Verwendung eines replizierenden retroviralen Konstruktes, wobei das replizierende virale Konstrukt ein replizierendes retrovirales Genom umfasst, zur Herstellung einer pharmazeutischen Zusammensetzung für die Gen-Zufuhr in Zellen in vivo, ex vivo oder in vitro für die prophylaktische oder therapeutische Behandlung eines Säugetiers und wobei das replizierende virale Konstrukt ein replizierendes retro-virales Genom umfasst, das in 5'- → 3'-Richtung die folgenden Elemente:
- eine retrovirale 5'-LTR,
- eine retrovirale Verpackungssequenz,
- funktionale gag-, pol- und env-Gene,
- ein Polynukleotid und
- ein retrovirales 3'-LTR,
wobei das retrovirale Genom zwischen dem env-Gen und dem Polynukleotid eine Spleiß-Akzeptorstelle aufweist.

2. Verwendung nach Anspruch 1, wobei das replizierende retrovirale Genom für eine retrovirale Hülle kodiert, die einen Tropismus für Säugetier-Zellen aufweist.

3. Verwendung nach Anspruch 2, wobei das Genom für ein modifiziertes Hüll-Glycoprotein kodiert.

4. Verwendung nach einem der Ansprüche 1 bis 3, wobei das Polynukleotid unter der transkriptionellen Kontrolle eines regulierten Promotors steht.

5. Verwendung nach Anspruch 3, wobei das modifizierte Hüll-Glycoprotein ein ausgewähltes Epitop umfasst.

6. Verwendung nach Anspruch 3, wobei das modifizierte Hüll-Glycoprotein einen modifizierten Wirtsbereich aufweist.

7. Verwendung nach einem der Ansprüche 1 bis 6, wobei das virale Konstrukt eine modifizierte LTR-Region aufweist, die in Gegenwart eines aktivierenden Polypeptids aktiv ist, und wobei das virale Konstrukt ein Polynukleotid umfasst, das für das aktivierende Polypeptid kodiert.

8. Verwendung nach einem der Ansprüche 1 bis 6, wobei das Konstrukt ein Polynukleotid umfasst, das für αGAL4 kodiert.

9. Verwendung nach einem der Ansprüche 1 bis 8, wobei das virale Konstrukt ein Plasmid oder ein defekter adenoviraler Vektor ist.

10. Pharmazeutische Zusammensetzung umfassend ein replizierendes retrovirales Konstrukt oder Genom, wobei das replizierende retrovirale Konstrukt oder Genom in 5'- → 3'-Richtung die folgenden Elemente umfasst:
- eine retrovirale 5'-LTR,
- eine retrovirale Verpackungssequenz,
- funktionale gag-, pol- und env-Gene,
- ein Polynukleotid und
- ein retrovirales 3'-LTR,
wobei das retrovirale Genom zwischen dem env-Gen und dem Polynukleotid eine Spleiß-Akzeptorstelle aufweist und die kodierte retrovirale Hülle Tropismus für Säugetierzellen aufweist.

11. Die pharmazeutische Zusammensetzung nach Anspruch 10, wobei das Genom für ein modifiziertes Hüll-Glycoprotein kodiert.

12. Die pharmazeutische Zusammensetzung nach einem der Ansprüche 10 bis 11, wobei das Polynukleotid unter der transkriptionellen Kontrolle eines regulierten Promotors steht.

13. Die pharmazeutische Zusammensetzung nach Anspruch 11, wobei das modifizierte Hüll-Glycoprotein ein ausgewähltes Epitop umfasst.

14. Die pharmazeutische Zusammensetzung nach Anspruch 11, wobei das modifizierte Hüll-Glycoprotein einen modifizierten Wirts-Bereich aufweist.

15. Die pharmazeutische Zusammensetzung nach einem der Ansprüche 10 bis 14, wobei das virale Konstrukt eine modifizierte LTR-Region aufweist, die in Gegenwart eines aktivierenden Polypeptids aktiv ist, und wobei das virale Konstrukt ein Polynukleotid umfasst, das für das aktivierende Polypeptid kodiert.

16. Die pharmazeutische Zusammensetzung nach einem der Ansprüche 10 bis 14, wobei das Konstrukt ein Polynukleotid umfasst, das für αGAL4 kodiert.

17. Rekombinantes replizierendes retrovirales Genom umfassend ein Polynukleotid eingefügt außerhalb der LTR-Sequenz und kodierend für eine retrovirale Hülle mit Tropismus für eine Säugetierzelle, wobei das virale Konstrukt eine modifizierte LTR-Region umfasst, die in Gegenwart eines aktivierenden Polypeptids aktiv ist, und wobei das virale Konstrukt ein Polynukleotid umfasst, das für das aktvierende Polypeptid kodiert.

18. Rekombinantes replizierendes retrovirales Genom umfassend ein Polynukleotid eingefügt außerhalb der LTR-Sequenz und kodierend für eine retrovirale Hülle mit Tropismus für eine Säugetierzelle, wobei das Konstrukt ein Polynukleotid umfasst, das für αGAL4 kodiert.

19. Das rekombinante replizierende retrovirale Genom nach Anspruch 17 oder 18, wobei das Polynukleotid unterhalb des env-Gens eingefügt ist, und wobei eine Spleiß-Akzeptorstelle zwischen dem env-Gen und dem Polynukleotid eingefügt ist.

20. Replizierendes Retrovirus, wobei das replizierende Retrovirus ein retrovirales Genom oder Konstrukt nach einem der Ansprüche 17 bis 19 umfasst.

21. Plasmid, wobei das Plasmid ein replizierendes retrovirales Genom, insbesondere ein replizierendes retrovirales Genom oder Konstrukt nach einem der Ansprüche 17 bis 19 umfasst.

22. Zusammensetzung umfassend ein Retrovirus nach Anspruch 20 oder ein Plasmid nach Anspruch 21.

23. Verfahren zum Versorgen einer Zelle, eines Gewebes oder eines Organs, in vitro oder ex vivo, mit einem Polynukleotid, wobei das Verfahren den Schritt umfasst, die Zelle, das Gewebe oder das Organ mit einer Zusammensetzung nach Anspruch 22 oder mit einer Retrovirus-Verpackungszelle in Berührung zu bringen, wobei diese Zelle, integriert in ihr Genom, ein replizierendes rekombinantes retrovirales Genom nach einem der Ansprüche 17 bis 19 umfasst.

24. Verwendung der Zusammensetzung nach Anspruch 22 oder einer Retrovirus-Verpackungszelle, die, integriert in ihr Genom, das replizierende rekombinante retrovirale Genom nach einem der Ansprüche 17 bis 19 umfasst, für die Herstellung einer Zusammensetzung, um eine Zelle, ein Gewebe oder ein Organ in vivo, ex vivo oder in vitro mit einem Polynukleotid zu versorgen.

25. Verwendung eines replizierenden retroviralen Konstrukts für die Herstellung einer pharmazeutischen Zusammensetzung, um Zellen in vivo, ex vivo oder in vitro mit einem Gen zu versorgen, für die prophylaktische oder therapeutische Behandlung von Säugetieren, wobei das replizierende virale Konstrukt ein replizierendes retrovirales Genom umfasst, das ein Polynukleotid umfasst, eingefügt außerhalb der LTR-Sequenz, und, in 5'- → 3'-Richtung die folgenden Elemente umfasst:
- eine retrovirale 5'-LTR,
- eine retrovirale Verpackungssequenz,
- funktionale gag-, pol- und env-Gene, und
- ein retrovirales 3'-LTR,
wobei das retrovirale Konstrukt ein Plasmid oder ein defekter adenoviraler Vektor ist.

26. Verwendung eines replizierenden retroviralen Konstrukts für die Herstellung einer pharmazeutischen Zusammensetzung, um Zellen in vivo, ex vivo oder in vitro mit einem Gen zu versorgen, für die prophylaktische oder therapeutische Behandlung von Säugetieren, wobei das replizierende virale Konstrukt ein replizierendes retrovirales Genom umfasst, das ein Polynukleotid umfasst, eingefügt außerhalb der LTR-Sequenz, und, in 5'- → 3'-Richtung die folgenden Elemente umfasst:
- eine retrovirale 5'-LTR,
- eine retrovirale Verpackungssequenz,
- funktionale gag-, pol- und env-Gene, und
- ein retrovirales 3'-LTR,
wobei das virale Konstrukt eine modifizierte LTR-Region umfasst, die in Gegenwart eines aktivierenden Polypeptids aktiv ist, und wobei das virale Konstrukt ein Polynukleotid umfasst, das für das aktivierende Polypeptid kodiert.

## Revendications

1. Utilisation d'une construction rétrovirale réplicative, ladite construction virale réplicative comprenant un génome rétroviral réplicatif, pour la préparation d'une composition pharmaceutique destinée à délivrer un gène dans des cellules in vivo, ex vivo, ou in vitro pour le traitement prophylactique ou thérapeutique d'un sujet mammifère, ladite construction virale réplicative comprenant dans le sens 5' -> 3' :
- un 5'-LTR rétroviral ;
- une séquence d'encapsidation rétrovirale ;
- des gènes gag, pol et env fonctionnels ;
- un polynucléotide ; et
- un 3'-LTR rétroviral,
le génome rétroviral comprenant un site accepteur d'épissage entre ledit gène env et ledit polynucléotide.

2. Utilisation selon la revendication 1, dans laquelle ledit génome rétroviral réplicatif code pour une enveloppe rétrovirale ayant un tropisme pour une cellule mammifère.

3. Utilisation selon la revendication 2, dans laquelle ledit génome code pour une glycoprotéine d'enveloppe modifiée.

4. Utilisation selon l'une quelconque des revendications 1 à 3, dans laquelle le polynucléotide est sous le contrôle transcriptionnel d'un promoteur régulé.

5. Utilisation selon la revendication 3, dans laquelle la glycoprotéine d'enveloppe modifiée comprend un épitope sélectionné.

6. Utilisation selon la revendication 3, dans laquelle la glycoprotéine d'enveloppe modifiée a un spectre d'hôtes modifié.

7. Utilisation selon l'une quelconque des revendications 1 à 6, dans laquelle ladite construction virale comprend une région LTR modifiée qui est active en présence d'un polypeptide activateur, et dans laquelle ladite construction virale comprend un polynucléotide codant ledit polypeptide activateur.

8. Utilisation selon l'une quelconque des revendications 1 à 6, dans laquelle ladite construction comprend un polynucléotide codant αGAL4.

9. Utilisation selon l'une quelconque des revendications 1 à 8, dans laquelle ladite construction virale est un plasmide ou un vecteur adénoviral défectif.

10. Composition pharmaceutique comprenant une construction ou un génome rétroviral réplicatif, dans laquelle ladite construction ou ledit génome rétroviral réplicatif comprend dans le sens 5' -> 3' :
- un 5'-LTR rétroviral ;
- une séquence d'encapsidation rétrovirale ;
- des gènes gag, pol et env fonctionnels ;
- un polynucléotide ; et
- un 3'-LTR rétroviral,
le génome rétroviral comprenant un site accepteur d'épissage entre ledit gène env et ledit polynucléotide, et l'enveloppe rétrovirale codée ayant un tropisme pour une cellule mammifère.

11. Composition pharmaceutique selon la revendication 10, dans laquelle ledit génome code pour une glycoprotéine d'enveloppe modifiée.

12. Composition pharmaceutique selon l'une quelconque des revendications 10 à 11, dans laquelle ledit polynucléotide est sous le contrôle transcriptionnel d'un promoteur régulé.

13. Composition pharmaceutique selon la revendication 11, dans laquelle la glycoprotéine d'enveloppe modifiée comprend un épitope sélectionné.

14. Composition pharmaceutique selon la revendication 11, dans laquelle la glycoprotéine d'enveloppe modifiée a un spectre d'hôtes modifié.

15. Composition pharmaceutique selon l'une quelconque des revendications 10 à 14, dans laquelle ladite construction virale comprend une région LTR qui est active en présence d'un polypeptide activateur, et dans laquelle ladite construction virale comprend un polynucléotide codant ledit polypeptide activateur.

16. Composition pharmaceutique selon l'une quelconque des revendications 10 à 14, dans laquelle ladite construction comprend un polynucléotide codant αGAL4.

17. Génome rétroviral réplicatif recombinant comprenant un polynucléotide inséré en dehors de la séquence LTR et codant une enveloppe rétrovirale ayant un tropisme pour une cellule mammifère, dans lequel ladite construction virale comprend une région LTR modifiée qui est active en présence d'un polypeptide activateur, et dans lequel ladite construction virale comprend un polynucléotide codant ledit polypeptide activateur.

18. Génome rétroviral réplicatif recombinant comprenant un polynucléotide inséré en dehors de la séquence LTR et codant une enveloppe rétrovirale ayant un tropisme pour une cellule mammifère, dans lequel ladite construction comprend un polynucléotide codant αGAL4.

19. Génome rétroviral réplicatif recombinant selon la revendication 17 ou 18, dans lequel le polynucléotide est inséré en aval du gène env et dans lequel un site accepteur d'épissage est inséré entre ledit gène env et ledit polynucléotide.

20. Rétrovirus réplicatif, dans lequel ledit rétrovirus réplicatif comprend un génome ou une construction rétroviral selon l'une quelconque des revendications 17 à 19.

21. Plasmide, dans lequel ledit plasmide comprend un génome rétroviral réplicatif, en particulier un génome ou une construction rétroviral selon l'une quelconque des revendications 17 à 19.

22. Composition comprenant un rétrovirus selon la revendication 20 ou un plasmide selon la revendication 21.

23. Méthode pour délivrer un polynucléotide à une cellule, un tissu ou un organe, in vitro ou ex vivo, comprenant la mise en contact de ladite cellule, dudit tissu ou dudit organe avec une composition selon la revendication 22 ou avec une cellule d'encapsidation de rétrovirus comprenant, intégré dans son génome, un génome rétroviral réplicatif recombinant selon l'une quelconque des revendications 17 à 19.

24. Utilisation d'une composition selon la revendication 22 ou d'une cellule d'encapsidation de rétrovirus comprenant, intégré dans son génome, un génome rétroviral réplicatif recombinant selon l'une quelconque des revendications 17 à 19 pour la préparation d'une composition destinée à délivrer un polynucléotide dans une cellule, un tissu ou un organe, in vivo, ex vivo ou in vitro.

25. Utilisation d'une construction rétrovirale réplicative pour la préparation d'une composition pharmaceutique destinée à délivrer un gène dans des cellules in vivo, ex vivo ou in vitro pour le traitement prophylactique ou thérapeutique d'un sujet mammifère, ladite construction rétrovirale réplicative comprenant un génome rétroviral réplicatif comprenant un polynucléotide inséré en dehors de la séquence LTR et, dans le sens 5' -> 3', :
- un 5'-LTR rétroviral ;
- une séquence d'encapsidation rétrovirale ;
- des gènes gag, pol et env fonctionnels ; et
- un 3'-LTR rétroviral,
ladite construction rétrovirale étant un plasmide ou un vecteur adénoviral défectif.

26. Utilisation d'une construction rétrovirale réplicative pour la préparation d'une composition pharmaceutique destinée à délivrer un gène dans des cellules in vivo, ex vivo ou in vitro pour le traitement prophylactique ou thérapeutique d'un sujet mammifère, ladite construction rétrovirale réplicative comprenant un génome rétroviral réplicatif comprenant un polynucléotide inséré en dehors de la séquence LTR et, dans le sens 5' -> 3', :
- un 5'-LTR rétroviral ;
- une séquence d'encapsidation rétrovirale ;
- des gènes gag, pol et env fonctionnels ; et
- un 3'-LTR rétroviral,
ladite construction virale comprenant une région LTR modifiée qui est active en présence d'un polypeptide activateur, et ladite construction virale comprenant un polynucléotide codant ledit polypeptide activateur.
